# EUROPEAN PATENT SPECIFICATION

(11) **EP 4 565 313 B1**
(45) Date of publication and mention of the grant of the patent: **10.06.2026**
(21) Application number: 23758380.2
(22) Date of filing: 27.07.2023
(51) Int. Cl.: A61N 1/36

(54) **AN AUTOMATIC DEVICE FOR TREATING THE SCALP**
AUTOMATISCHE VORRICHTUNG ZUR BEHANDLUNG DER KOPFHAUT
DISPOSITIF AUTOMATIQUE POUR LE TRAITEMENT DU CUIR CHEVELU

(30) Priority: 03.08.2022 IT 202200016449
(43) Date of publication of application: 11.06.2025
(73) Proprietor: APS S.r.l., 48018 Faenza (RA) (IT)
(72) Inventor: NATI, Daniele, 48018 Faenza (RA) (IT)
(74) Representative: Dall'Olio, Christian
(86) International application number: PCT/IB2023/057613
(87) International publication number: WO 2024/028706

(56) References cited:
- KR-A- 20210 091 842
- US-A1- 2014 330 196
- US-A1- 2018 015 299
- US-A1- 2022 160 576

## Description

### FIELD OF THE INVENTION

The present invention relates to the technical sector concerning automatic devices for the treatment of pathologies of the scalp.

In a healthy scalp up to 3 hairs can coexist in each hair follicle (or bulb). The scalp can be subject to various pathologies, some of which can also lead to hair thinning or the loss of hairs and/or a thinning of the relative shaft. Hair thinning often brings about the presence of a single hair in the hair bulbs, typically having a narrowed shaft with respect to a normal hair.

Among other alterations, anomalies and pathologies related to the scalp and its adnexa can be included: telogen effluvium, hair thinning, alopecia, baldness, androgenic alopecia, seborrheic dermatitis, dandruff, hyperseborrhea, hyperhydrosis, psoriasis, itching, trichodynia (scalp pain), etc.

Obviously pathologies of the scalp involving the physical aspect have a significant effect on an individual's life, for which reason there has always been an intent to treat them using pharmacological and physical treatments, and sometimes both. This is possible when the hair follicles are still active, i.e. there is still the possibility of generating hairs even if they are partly atrophised, and/or have one of two hairs even if from a shaft that is reduced in size.

Androgenetic alopecia (AGA) is the most common cause of non-scarring alopecia, which strike respectively up to 50% of women and up to 80% of men during the course of their lives, with a frequency that increases with age after puberty. The disease is characterised by a progressive miniaturisation of the hair follicles in a selective distribution at the top and in the frontal area of the head, in genetically predisposed individuals.

### DESCRIPTION OF THE PRIOR ART

Trichological pharmacological compositions for cutaneous use are known and are for application on the area to be treated by slow massaging. Their therapeutic effectiveness is such as not to guarantee complete healing and to provide, for less serious cases, relatively long improvement times. For this reason efforts have been made to increase the absorption by the scalp of these compositions. By way of example, among the trichological active ingredients can also be included the following: ketoconazole, selenium sulphide, zinc pyrithione, sulphur and corticosteroids as well as growth factors based on finasteride (Propecia) or on Minoxidil (Rogaine).

Italian patent IT 102018000003548 belonging to the present Applicant described the use of a electrically-conductive pharmacological composition and an automatic device for treating the scalp having a first operating head comprising a plurality of vibrating protuberances and a plurality of electrodes, wherein each electrode of the plurality of electrodes projects distally from a different protuberance of the plurality of protuberances. The head includes, as well as the vibration of the protuberances, also the possibility of electrostimulating the scalp and facilitating the absorption of the trichologically active ingredient present in the conductive pharmacological composition by means of iontopheresis. However the effectiveness of the vibration on the vascularisation of the scalp is low. Therefore, the device is used after treatment with the operating head of an automatic device for microdermabrasion of the skin, according to Italian patent IT 102015000009009. The micro-dermoabrasive head has a plurality of perforating spikes which project from a corresponding plurality of truncated cones. Once the head is activated in such a way as to stretch the scalp while it is being repeatedly perforated with the perforating spikes while the surface of the truncated cone strikes and stretches it.

The treatment carried out by the head according to IT 102015000009009 followed by the treatment carried out with the head according to IT 102018000003548 is clearly very effective, but as it includes the micro-dermoabrasion of the scalp, it is somewhat uncomfortable for the patient and can be carried out only by medical personnel in appropriate therapeutic sessions, with consequent relatively high costs. Further, as the micro-dermoabrasion implies loss of blood the medical personnel must take utmost care during the entire treatment so as to avoid contact with the blood. It is noteworthy that it is necessary to carry out at least one change of head during the treatment. There is also a perceived need to reduce the number of therapeutic sessions in proportion to the obtainable result. Additionally, the need arises to be able to carry out further treatments without any need to involve medical personnel, between one therapeutic session and the next, or to prolong the effect after the end of the sittings.

Platelet-rich plasma (PRP), which contains growth factors, has been researched in the literature as an injectable trichological composition to facilitate hair growth in patients subject to hair loss. Numerous studies have shown that PRP injections represent a therapeutic option for androgenetic alopecia, with good general levels of satisfaction on the part of the patient. However, treatment with PRP is a complex procedure as the patient's blood must be collected at each session so as to produce autologous PER to be injected and leads to hospital refuse, management of which is complicated and the disposal cost of which is high. Moreover, this treatment requires special equipment for production of PRP and this production is difficult to standardise. Consequently, it is arduous to stabilise the effective dose, the duration of the treatment and the long-term security profile. Additionally, treatment with RPR is an expensive procedure.

US2014330196 discloses an automatic device for treating the scalp according to the preamble of claim 1.

### SUMMARY OF THE INVENTION

The main aims of the present invention are the halting or reversing of the miniaturisation of hairs (reduction of the diameter of the shaft) and the prolongation of the anagen phase, with the aim of normalising the follicular cycle.

A further aim of the present invention is to reduce the need for trichological sessions carried out by medical personnel while at the same time guaranteeing high trichological effectiveness.

In particular, an objective of the present invention consists in reducing the number of trichological sessions carried out by medical personnel or increasing the interval between one and the next by means of trichological treatments which can be carried out by non-medical personnel, for example hairdressers, beauty salon operatives, etc., or which can be carried out by the patient him or herself at home. Obviously, making these trichological treatments available for use by non-medical personnel is a further aim of the present invention.

The above-mentioned aim has been obtained with the automatic device according to claim 1.

The automatic device for treating the scalp comprises:
- a fixed portion, in turn comprising: a casing; a first direct current electricity generator, activatable to generate a direct current and arranged in the casing; a second voltage generator, which is arranged in the casing and which is activatable to generate a current having a biphasic rectangular or triangular waveform, having a frequency comprised between 2.0 and 3.0 Hz;
- a handle electrically connected to the first direct current electricity generator and to the second voltage generator and, in turn, comprising: a body; a motor having a drive shaft, both arranged in the body with the motor being arranged proximally and with the drive shaft being arranged distally and longitudinally with respect to the body, a motion transmission element which is solidly constrained to a distal end of the drive shaft and which has a motion transmission surface which is distal and which is inclined, with respect to the drive shaft, by a minimum angle comprised between 5° and 85°, the motor being electrically activatable to move the motion transmission surface in rotation with respect to the drive shaft; a first electrical contact; a second electrical contact;
- at least a lighting device, activatable to emit a coherent non-collimated monochromatic electromagnetic radiation;
- an operating head comprising: a first plurality of rounded protuberances made of an electrically conductive material, a second plurality of rounded protuberances made of an electrically conductive material, a third electrical contact, electrically connected to the first plurality of protuberances; a fourth electrical contact electrically connected to the second plurality of rounded protuberances; a piston-cylinder system, in turn comprising, a tubular element which functions as a cylinder of the piston-cylinder system and which has a distal end; a piston which: has a rod and a head having a distal end, wherein the piston is slidably inserted in the tubular element with freedom to move from a first limit position, wherein the distal end of the head is arranged externally of the tubular element and at a first distance, measured parallel to the rod, from a distal end of the tubular element, to a second limit position, wherein the distal end of the head is arranged at a second distance, measured parallel to the rod, from the distal end of the tubular element, that is less than the first distance; or wherein the distal end of the head is arranged internally of the tubular element; and elastic means acting on the head and predisposed to automatically move the piston from the first limit position to the second limit position; wherein the first and second plurality of rounded protuberances are solidly constrained to the distal end of the head and project distally therefrom;

wherein the at least a lighting device is solidly constrained to the handle and arranged distally or is solidly constrained to the operating head and distally arranged;
wherein the tubular element is solidly and reversibly fixable to the handle in such a way that the operating head is at least partially internal of the body of the handle and supported by the handle electrically connecting the first electrical contact to the third electrical contact and the second electrical contact to the fourth contact, arranging the rod offset, but substantially parallel to the drive shaft, and in contact with the motion transmission surface and arranging the distal end of the head externally of the handle, when in the first limit position, and wherein the at least a lighting device, the motor, the first generator, and the second voltage generator, are activatable contemporaneously.

Note that, with the motor activated, the motion transmission surface (7) together with the elastic means (14) move the piston with a straight alternating movement, wherein the motion transmission surface (7) moves the piston from the second limit position to the first limit position and the elastic means (14) from the first limit position to the second limit position.

The fact that according to the invention the first and second plurality of rounded protuberances is solidly constrained to the head of the piston significantly increases the effectiveness in vascularising the scalp with respect to the operating head of Italian patent IT 102015000009009. This also leads to greater absorption of the trichologically active ingredient and, in general, a greater effectiveness of the combined treatment of mechanical stimulation, electrostimulation, electrophoresis and radiation with a consequent improvement of the diameter of the hair shaft and the number of hairs per hair follicle, with respect to the operating head of patent IT 102015000009009.

It is clear that the particular conformation of the rounded protuberances of the automatic device for treating the scalp avoids excoriations of the scalp and loss of blood and therefore this device can also be used by non-medical personnel.

The applicant has found that a treatment of only 15 minutes, repeated over time, has a significant effectiveness that is greater than what is obtainable according to IT 102015000009009.

The term "substantially parallel to the drive shaft" is taken to mean parallel to the drive shaft or inclined, with respect to the drive shaft, by an angle of less than 3°, preferably less than 2°, more preferably less than 1°, still more preferably less than 0.8°, advantageously less than 0.5°, more advantageously less than 0.3°, still more advantageously less than 0.2° or 0.1°.

### BRIEF DESCRIPTION OF THE DRAWINGS:

Specific embodiments of the invention will be described in the following part of the present description, according to what is set down in the claims and with the aid of the accompanying tables of drawings, in which:
- figure 1 is a perspective view of an automatic device for treating scalp pathologies according to the invention;
   figure 2 is a frontal view of the automatic device of figure 1;
   figure 3 is a rear view of the automatic device of figure 1;
   figure 4 is a view from above of a handle of the automatic device according to the invention;
   figure 5 is a lateral view of the handle of figure 4;
   figure 6 is a frontal view of the handle of figure 4;
   figure 7 is a rear view of the handle of figure 4;
   figure 8 is a perspective view of the handle of figure 4;
   figure 9 is a lateral and partially exploded view of the handle of figure 4;
   figure 10 is a further lateral and partially exploded view of the handle of figure 4;
   figure 11 is a lateral view of the operating head of the handle of figure 4 and of the motor of the handle in a first operating arrangement.
   figure 12 is a lateral view of the operating head and of the motor of figure 11 in a second operating arrangement.
   figure 13 is a perspective and partially exploded view of the handle of figure 4;
   figure 14 is a frontal view of the operating head of figure 11;
   figure 15 is a view from above of the operating head of figure 11;
   figure 16 is a section view along plane XVI-XVI of figure 15 of the operating head of figure 11;
   figure 17 is a lateral view of the operating head of figure 11;
   figure 18 is a section view along plane XVIII-XVIII of figure 17 of the operating head of figure 11;
   figure 20 is a top exploded view of the operating head of figure 11;
   figure 21 is a lateral exploded view of the operating head of figure 11; and
   figure 22 is a frontal view of the exploded view of figure 21.

Note that in these figures only the mechanical part is represented of what is illustrated, while the electrical part has been omitted for reasons of clarity.

### DESCRIPTION OF PREFERRED EMBODIMENTS

With reference to the appended figures of the drawings, an automatic device for treating the scalp is denoted by reference numeral 1, comprising:
- a fixed portion (2), in turn comprising: a casing; a first direct current electricity generator, activatable to generate a direct current and arranged in the casing; a second voltage generator, which is arranged in the casing and which is activatable to generate a current having a biphasic rectangular or triangular waveform, having a frequency comprised between 2.0 and 3.0 Hz;
- a handle (3) electrically connected to the first direct current electricity generator and to the second voltage generator and, in turn, comprising: a body; a motor (4) having a drive shaft (5), both arranged in the body with the motor (4) being arranged proximally and with the drive shaft (5) being arranged distally and longitudinally with respect to the body, a motion transmission element (6) which is solidly constrained to a distal end of the drive shaft (5) and which has a motion transmission surface (7) which is distal and which is inclined with respect to the drive shaft (5), by a minimum angle (α) comprised between 5° and 85°, the motor (4) being electrically activatable to move the motion transmission surface (7) in rotation with respect to the drive shaft (5); a first electrical contact; a second electrical contact;
- at least a lighting device (8), activatable to emit a coherent non-collimated monochromatic electromagnetic radiation;
- an operating head comprising: a first plurality of rounded protuberances (10) made of an electrically conductive material, a second plurality of rounded protuberances (10) made of an electrically conductive material, a third electrical contact, electrically connected to the first plurality of protuberances; a fourth electrical contact electrically connected to the second plurality of rounded protuberances (10; a piston-cylinder system, in turn comprising, a tubular element (11) which functions as a cylinder of the piston-cylinder system and which has a distal end; a piston which: has a rod (12) and a head (13) having an distal end, wherein the piston is slidably inserted in the tubular element (11) with freedom to move from a first limit position, wherein the distal end of the head (13) is arranged externally of the tubular element (11) and at a first distance (D1), measured parallel to the rod, from a distal end of the tubular element, to a second limit position, wherein the distal end of the head (13) is arranged at a second distance (D2), measured parallel to the rod, from the distal end of the tubular element, that is less than the first distance (D1); or wherein the distal end of the head (13) is arranged internally of the tubular element (11); and elastic means (14) acting on the head (13) and predisposed to automatically move the piston from the first limit position to the second limit position; wherein the first and second plurality of rounded protuberances (10) are solidly constrained to the distal end of the head and project distally therefrom;

wherein the at least a lighting device (8) is solidly constrained to the handle (3) and arranged distally or is solidly constrained to the operating head and distally arranged;
wherein the tubular element (11) is solidly and reversibly fixable to the handle (3) in such a way that the operating head is at least partially internal of the body of the handle (3) and supported by the handle (3) electrically connecting the first electrical contact to the third electrical contact and the second electrical contact to the fourth contact, arranging the rod offset, but substantially parallel to the drive shaft (5), and in contact with the motion transmission surface (7) and arranging the distal end of the head (13) externally of the handle (3), when in the first limit position. This allows, when the operating head is supported by the handle (3), with the first and second plurality of rounded protuberances (10) in contact with the scalp covered by an electrically conductible composition comprising a trichologically active ingredient, and the at least a lighting device (8) and the motor (4) are activated, to illuminate the scalp while a first direct electric current is transmitted to the scalp with the purpose of facilitating the absorption of the trichologically active ingredient by the scalp and electrostimulating the scalp by means of a second current generated by the second voltage generator while the motion transmission surface (7) together with the elastic means (14) move the piston with a straight alternating movement, wherein the motion transmission surface (7) moves the piston from the second limit position to the first limit position and the elastic means (14) from the first limit position to the second limit position. Obviously, to do this, it is clear that the at least a lighting device (8), the motor (4), the first generator and the second voltage generator are also activatable contemporaneously with one another.

Note that, when the motor is activated, the motion transmission surface (7) together with the elastic means (14) move the piston with a straight alternating movement, wherein the motion transmission surface (7) moves the piston from the second limit position to the first limit position and the elastic means (14) from the first limit position to the second limit position.

The wave form is preferably rectangular with a frequency of 2.5 Hz, as this enables obtaining a greater effectiveness of the trichological treatment performed with the automatic device (1) of the invention.

Obviously the motion transmission surface (7) will be inclined with respect to the drive shaft (5), even by a maximum angle not illustrated, supplementary to the minimum angle (α).

Alternatively, the biphasic current has a wave form that is rectangular, having a frequency comprised between 2.2 and 3, more preferably between 2.4 and 2.6 Hz. The wave form is preferably rectangular with a frequency of 2.5 Hz.

The at least a lighting device (8) can advantageously be a LED. The at least a lighting device (8) can preferably emit a luminous monochromatic electromagnetic radiation having a wavelength of 630 nm (red), 415 nm (blue) or 850 nm (green).

The at least a lighting device (8) is advantageously a plurality of LEDs able to emit a light having a wavelength of 630, 415, or 850 nm, preferably of 630 and/or 415, more preferably 630 nm.

In an embodiment of the invention, the at least a lighting device (8) can comprise a plurality of lighting devices comprising at least the lighting device emitting a blue luminous coherent monochromatic radiation, at least a lighting device (8) emitting a red luminous coherent monochromatic radiation, at least a lighting device (8), emitting a respective green luminous coherent monochromatic radiation.

In accord to a preferred embodiment of the automatic device (1) of the invention, the at least a lighting device (8) is solidly constrained to the handle (3) and electrically connected to the first direct current electricity generator and arranged upstream of the distal end (13) of the head and facing distally. This enables obtaining an operating head that is lighter and easier to realise with respect to an operating head wherein the at least a lighting device (8) is solidly constrained to the operating head. In this case it is preferable for the at least a lighting device (8) to be solidly constrained to the tubular element (11) rather than to the head (13) of the piston. This is so as to avoid alternating straight movement of the at least a lighting device.

With the aim of radiating a greater surface of scalp (3), the at least a lighting device (8) is preferably a plurality of lighting devices arranged along a looped pathway. When the operating head is supported by the handle (3), the lighting device advantageously surrounds the piston.

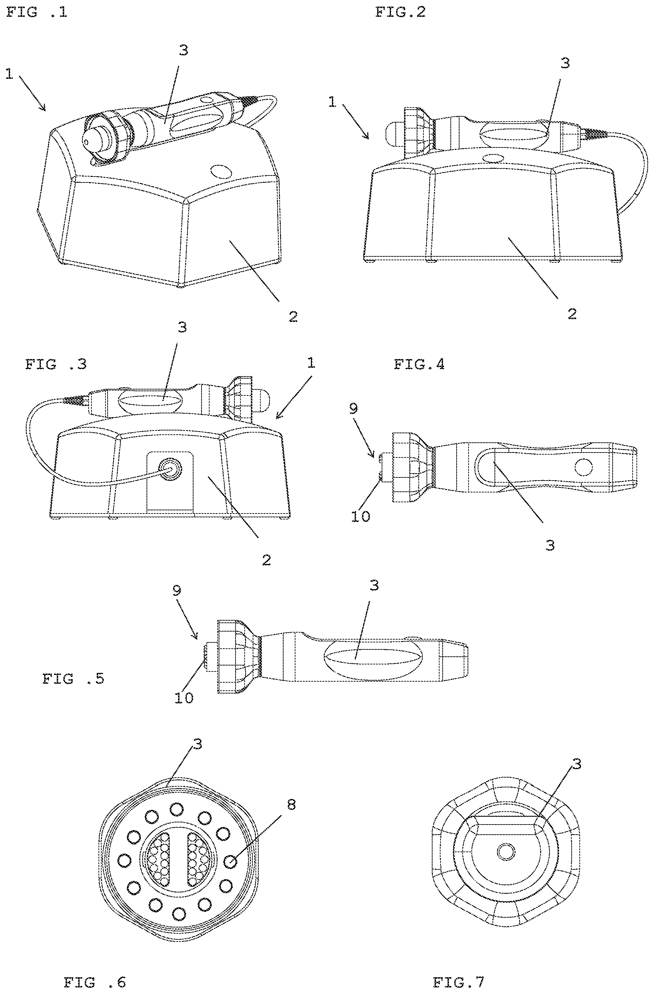

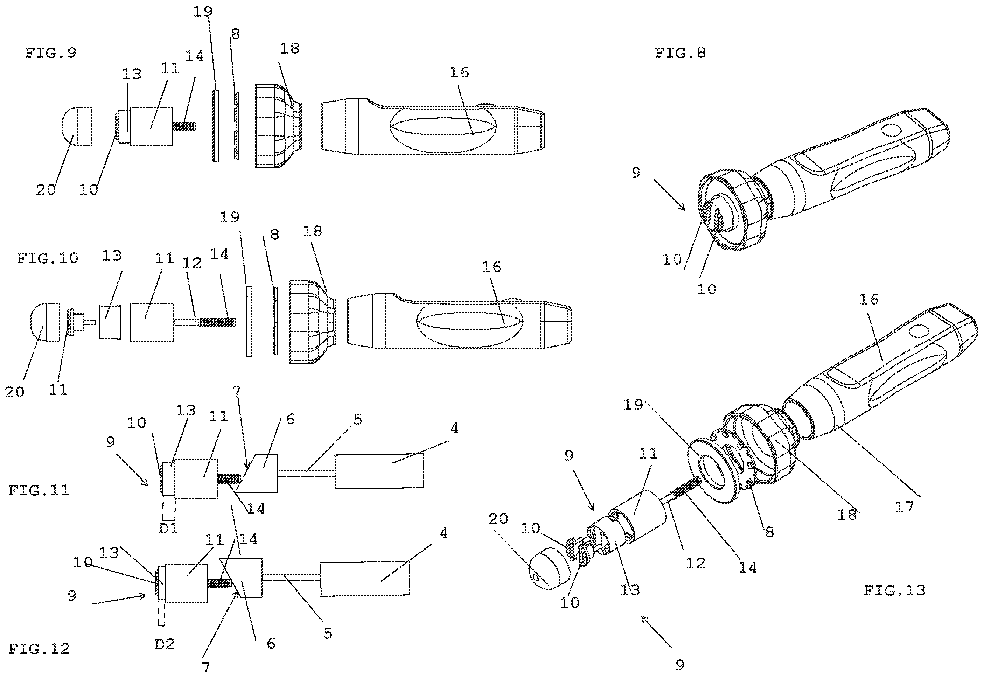

As illustrated in figures 1-10 and 13 the body of the handle (3) can comprise an elongate first proximal part (16) (see figures 9, 10 and 13) having a distal opening (17) and an annular and distal second part (18), which is couplable to the opening to form an annular flap the radial extension of which with respect to the longitudinal axis of the body of the handle is greater than that of the elongate first proximal part (16) of the body with the aim of maintaining a good handleability of the handle (3). According to this embodiment, the looped pathway is arranged along the annular and distal second part (18). A distal annular element (19) made of glass can be included to protect the at least a lighting device and a cover (20) to cover the first and the second plurality of rounded protuberances (10).

In a preferred aspect of the invention, each rounded protuberance (10) of the first and/or the second plurality of rounded protuberances (10) projects distally from the distal end of the head (13) by 0.1 - 15 mm, and then preferably progressively by 0.3 - 1.2 mm, 0.4 - 3 mm, 0.5 - 2.0 mm and preferably by 1 mm.

The motor (4) is preferably activatable with a rotation velocity of the drive shaft (5) of 5 - 100 revolutions per second, and then preferably progressively by 5-80, 10-50, 20-40, 20-50 revolutions per second.

In a preferred aspect of the invention, each rounded protuberance (10) of the first and/or the second plurality of rounded protuberances (10) is conformed as a hemisphere, a truncated cone with rounded corners or a cone with a bevelled tip.

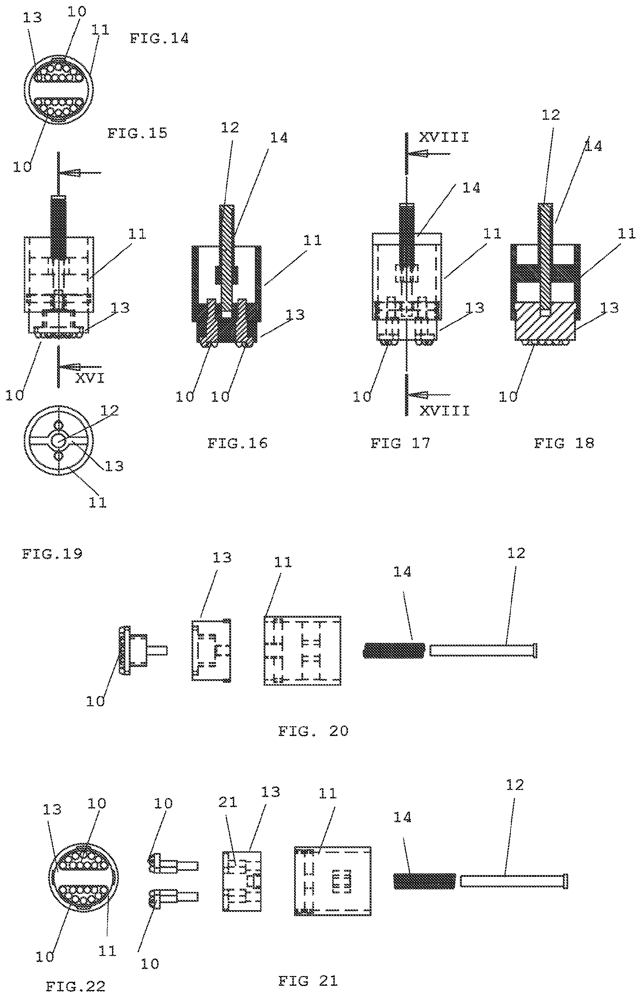

According to an illustrated first embodiment the distal end of the head (13) is made of an electrically insulated material and comprises a first and a second housing (21), separate from one another, respectively, with the first and second plurality of rounded protuberances (10) with the purpose of supporting them. In relation to this, see figure 21.

According to a preferred aspect of the invention the minimum angle (α) is preferably comprised between 15 and 70°, between 20° and 50°, between 30° and 45°. This enables obtaining a straight alternating movement of the piston with good elongation and good contact between the rod and the motion transmission surface (7).

According to the first embodiment illustrated, the elastic means (14) can advantageously be a spring wound about the rod and which extends from the head (13) to the motion transmission surface (7).

According to the invention, an automatic device (1) is preferred in which the at least a lighting device (8), the motor (4) and the second voltage generator are also activatable separately from one another and the first direct electric current can be generated even when the at least a lighting device (8), and/or the motor (4) and/or the second voltage generator are not activated. This allows personalisation of the trichological treatment on the basis of the specific needs of the patient.

### EXPERIMENTAL DATA

The automatic device (1) of the invention was used to treat androgenetic alopecia in both male and female patients, for a period of 6 months. The assessment was subjective, with the views of the operator and the patient, as well as objective, using the comparison of overall photographs and series micro photography (trichoscopy with enlargements of 20X, 40X, 70X).

### Materials and methods

20 patients were enrolled, 10 male and 10 female, in an age range of between 18 and 70 years, affected by grade **II, III, IV** and V androgenetic alopecia following the Hamilton scale for the males and grade **I, II, III** for females following the Ludwig scale.

Subjects excluded from the research were those affected by pre-cancerous conditions, neoplastic conditions or serious systemic pathologies (diabetes, cirrhosis); subjects under cosmetic or pharmacological treatment with Finasteride or Minoxidil; pregnant or nursing women. The study was conducted over six months overall.

During the first recruiting meeting (T0), the patients underwent dermatological examination, overall and micro photographic examination (trichoscopy) using Trichoscan^{®} (FotoFinder dermoscope, Teachscreen Software, Bad Birnbach, Germany).

Trichoscan^{®} carries out a mapping of an area of the scalp, taking into consideration the number of hairs present and the percentage of hairs affected by miniaturisation, which is the process identifying androgenetic alopecia.

This technique consists in photographing, over successive months, the same predetermined area, so that it is possible to track over time the changes in number and diameter of the hairs in response to the treatment.

Every 3 weeks, on 4 consecutive occasions, the volunteer was clinically assessed by the researcher. At the final examination after 6 months, the patient was newly reassessed using instrumental methodologies. The volunteer was further asked to fill a questionnaire on the effect of the treatment, the cosmetic appreciability of the product and its effectiveness.

During each check-up the patient was subjected to treatment, with topical application of a vial containing an electrically conductible composition comprising growth factors and thereafter a 15-minute treatment with the automatic device (1) according to the invention to facilitate absorption of the composition. The treatment included, contemporaneously, the straight alternating movement of the first and the second plurality of rounded protuberances (10), electrostimulation, emission of the first direct electric current and emission of a coherent non-collimated monochromatic electromagnetic radiation having a wavelength of 630 mn.

### RESULTS

All 20 patients finished the study without adverse reactions or side-effects.

### MALE PATIENTS

The trichoscopy evidenced an improvement in all 10 patients.

**Front area:** an increase was observed of 14.48% in the total number of hairs/area with a 1 cm² diameter (average t0: 1146.0, average t6: 1340.1) and a reduction by 6.58% of fuzz hair/area with a 1 cm² diameter (average t0: 466.7%, average t6: 437.90%). The diameter of the hair shaft increased by 8.45%.

**Area of the top of the head:** an increase was observed of 14.48% in the total numbers of hairs/area with a 1 cm² diameter (average t0: 1146.0, average t6: 1340.1) and a reduction by 6.58% of the hairs of the fuzz hair/area with a 1 cm² diameter (average t0: 466.7%, average t6: 437.90%). The diameter of the hair shaft increased by 8.45%.

### FEMALE PATIENTS

The trichoscopy evidenced an improvement in all 10 patients.

**Front area:** an increase was observed of 12.52% in the total number of hairs/area with a 1 cm² diameter (average t0: 1117.7, average t6: 1277.7) and a reduction by 34.39% of fuzz hair/area with a 1 cm² diameter (average t0: 489.6%, average t6: 364.3%). The diameter of the hair shaft increased by 15.61%.

**Area of the top of the head:** an increase was observed of 11.34% in the total number of hairs/area with a 1 cm² diameter (average t0: 796 average t6: 897.8) and a reduction by 26.31% of fuzz hair/area with a 1 cm² diameter (average t0: 350.5%, average t6: 277.5%). The diameter of the hair shaft increased by 16.55%.

### Researcher's assessment

At a distance of 6 months, the researcher's assessment agreed with the effectiveness in all male patients: no patient was judged to be unstable, 4 with a moderate improvement and 6 with a significant improvement.

At a distance of 6 months, the researcher's assessment observed an excelled therapeutic effectiveness in female patients: 2 with a slight improvement, 2 with a moderate improvement and 6 with a significant improvement.

### Patient's assessment

The patient's assessment showed effectiveness in all male patients: 4 with a moderate improvement and 6 with a significant improvement.

The assessment of the female patients showed effectiveness as follows: 5 with a moderate improvement, 5 with a significant improvement.

The study revealed a significant improvement of the androgenetic alopecia in both male and female patients, with a reduction in the loss of hairs and an increased diameter of the hairs in all areas of the scalp treated.

Further, the applicant verified that only a few treatments (3 or 4) with the device according to the invention are sufficient to reduce triodynia (often eliminating it entirely) in patients with that condition.

The applicant found that the above-cited results are not obtainable with only use of the operating head for an automatic device (1) for transdermal vehiculation of an active ingredient, according to patent IT102018000003548.

It is understood that the above has been described by way of non-limiting example.

## Claims

1. An automatic device (1) for treating the scalp, comprising:
- a fixed portion (2), in turn comprising: a casing; a first direct current electricity generator, activatable to generate a direct current and arranged in the casing; a second voltage generator, which is arranged in the casing and which is activatable to generate a current having a biphasic rectangular or triangular waveform, having a frequency comprised between 2.0 and 3.0 Hz;
• - a handle (3) electrically connected to the first direct current electricity generator and to the second voltage generator and, in turn, comprising: a body; a motor (4) having a drive shaft (5); - at least a lighting device (8) solidly constrained to the handle (3) and arranged distally or is solidly constrained to the operating head and distally arranged; ;
wherein the automatic device (1) for treating the scalp is **characterized**:
• **in that** the at least a lighting device (8) is activatable to emit a coherent non-collimated monochromatic electromagnetic radiation;
• **in that** the motor (4) is arranged proximally and with the drive shaft (5) being arranged distally and longitudinally with respect to the body, a motion transmission element (6) which is solidly constrained to a distal end of the drive shaft (5) and which has a motion transmission surface (7) which is distal and which is inclined, with respect to the drive shaft (5), by a minimum angle (α) comprised between 5° and 85°, the motor (4) being electrically activatable to move the motion transmission surface (7) in rotation with respect to the drive shaft (5);
• **in that** the handle (3) further comprises: a motion transmission element (6) which is solidly constrained to a distal end of the drive shaft (5) and which has a motion transmission surface (7) which is distal and which is inclined, with respect to the drive shaft (5), by a minimum angle (α) comprised between 5° and 85°, the motor (4) being electrically activatable to move the motion transmission surface (7) in rotation with respect to the drive shaft (5); a first electrical contact; and a second electrical contact; and
• **in that** the automatic device (1) for treating the scalp, further comprises:
- an operating head comprising: a first plurality of rounded protuberances (10) made of an electrically conductive material, a second plurality of rounded protuberances (10) made of an electrically conductive material, a third electrical contact, electrically connected to the first plurality of protuberances; a fourth electrical contact electrically connected to the second plurality of rounded protuberances (10); a piston-cylinder system, in turn comprising, a tubular element (11) which functions as a cylinder of the piston-cylinder system and which has a distal end; a piston which: has a rod (12) and a head (13) having an distal end, wherein the piston is slidably inserted in the tubular element (11) with freedom to move from a first limit position, wherein the distal end of the head (13) is arranged externally of the tubular element (11) and at a first distance (D1), measured parallel to the rod, from a distal end of the tubular element, to a second limit position, wherein the distal end of the head (13) is arranged at a second distance (D2), measured parallel to the rod, from the distal end of the tubular element, that is less than the first distance (D1); or wherein the distal end of the head (13) is arranged internally of the tubular element (11); and elastic means (14) acting on the head (13) and predisposed to automatically move the piston from the first limit position to the second limit position; wherein the first and second plurality of rounded protuberances (10) are solidly constrained to the distal end of the operating head and project distally there from;
wherein the tubular element (11) is solidly and reversibly fixable to the handle (3) in such a way that the operating head is at least partially internal of the body of the handle (3) and supported by the handle (3) electrically connecting the first electrical contact to the third electrical contact and the second electrical contact to the fourth contact, arranging the rod offset, but substantially parallel to the drive shaft (5), and in contact with the motion transmission surface (7) and arranging the distal end of the head (13) externally of the handle (3), when in the first limit position, and wherein the at least a lighting device (8), the motor (4), the first generator, and the second voltage generator, are activatable contemporaneously.

2. The automatic device for treating the scalp of claim 1, wherein the at least a lighting device (8) is solidly constrained to the handle (3) and electrically connected to the first direct current electricity generator and arranged upstream of the distal end (13) of the head and facing distally.

3. The automatic device for treating the scalp of claim 2, wherein the at least a lighting device (8) is a plurality of lighting devices (8) arranged along a looped pathway, which, when the operating head is supported by the handle (3), surrounds the piston.

4. The automatic device for treating the scalp of any one preceding claim, wherein the at least a lighting device (8) is a plurality of LEDs able to emit a light having a wavelength of 630, 415, or 850 nm.

5. The automatic device for treating the scalp, of any one preceding claim, wherein each rounded protuberance (10) of the first and/or the second plurality of rounded protuberances (10) projects distally from the distal end of the head (13) by 0.1 - 15 mm.

6. The automatic device for treating the scalp of any one preceding claim, wherein the motor (4) is activatable with a rotation velocity of the drive shaft (5) of 5 - 100 revolutions per second.

7. The automatic device for treating the scalp, according to any one preceding claim, wherein each rounded protuberance (10) of the first and/or the second plurality of rounded protuberances (10) is conformed as a hemisphere, a truncated cone with rounded corners or a cone with a bevelled tip.

8. The automatic device for treating the scalp of any one preceding claim, wherein the distal end of the head (13) is made of an electrically insulated material and comprises a second housing, separate from one another, engageable respectively, with the first and second plurality of rounded protuberances (10) with the purpose of supporting them.

9. The automatic device for treating the scalp of any one preceding claim, wherein the minimum angle (α) is comprised between 20° and 50°.

10. The automatic device for treating the scalp of any one preceding claim, wherein the at least a lighting device (8), the motor (4), the second voltage generator are also activatable separately from one another and the first direct electric current can be generated even when the at least a lighting device (8), and/or the motor (4) and/or the second voltage generator are not activated.

## Patentansprüche

1. Automatische Vorrichtung (1) zur Behandlung der Kopfhaut, umfassend:
- einen feststehenden Teil (2), der seinerseits umfasst: ein Gehäuse; einen ersten Gleichstromgenerator, der zur Erzeugung eines Gleichstroms aktivierbar ist und in dem Gehäuse angeordnet ist; einen zweiten Spannungsgenerator, der in dem Gehäuse angeordnet ist und zur Erzeugung eines Stroms mit einer biphasischen Rechteck- oder Dreieckwellenform mit einer Frequenz zwischen 2,0 und 3,0 Hz aktivierbar ist;
- einen Griff (3), der elektrisch mit dem ersten Gleichstromgenerator und dem zweiten Spannungsgenerator verbunden ist und seinerseits umfasst: einen Körper; einen Motor (4) mit einer Antriebswelle (5); - mindestens eine Beleuchtungseinrichtung (8), die fest mit dem Griff (3) verbunden und distal angeordnet ist oder fest mit dem Arbeitskopf verbunden und distal angeordnet ist;
wobei die automatische Vorrichtung (1) zur Behandlung der Kopfhaut **dadurch gekennzeichnet ist**:
• **dass** die mindestens eine Beleuchtungseinrichtung (8) so betätigbar ist, dass sie eine kohärente, nicht kollimierte, monochromatische elektromagnetische Strahlung emittiert;
• **dass** der Motor (4) proximal angeordnet ist und, wobei die Antriebswelle (5) distal und in Längsrichtung in Bezug auf den Körper angeordnet ist, ein Bewegungsübertragungselement (6) vorgesehen ist, das fest mit einem distalen Ende der Antriebswelle (5) verbunden ist und eine Bewegungsübertragungsfläche (7) aufweist, die distal angeordnet und in Bezug auf die Antriebswelle (5) um einen Mindestwinkel (α) zwischen 5° und 85° geneigt ist, wobei der Motor (4) elektrisch aktivierbar ist, um die Bewegungsübertragungsfläche (7) relativ zur Antriebswelle (5) in Drehung zu versetzen;
• **dass** der Griff (3) ferner umfasst: ein Bewegungsübertragungselement (6), das fest mit einem distalen Ende der Antriebswelle (5) verbunden ist und eine distale Bewegungsübertragungsfläche (7) aufweist, die in Bezug auf die Antriebswelle (5) um einen Mindestwinkel (α) zwischen 5° und 85° geneigt ist, wobei der Motor (4) elektrisch aktivierbar ist, um die Bewegungsübertragungsfläche (7) relativ zur Antriebswelle (5) in Drehung zu versetzen; einen ersten elektrischen Kontakt; und einen zweiten elektrischen Kontakt; und
• **dass** die automatische Vorrichtung (1) zur Behandlung der Kopfhaut ferner umfasst:
- einen Arbeitskopf, der umfasst: eine erste Vielzahl von abgerundeten Vorsprüngen (10) aus einem elektrisch leitfähigen Material, eine zweite Vielzahl von abgerundeten Vorsprüngen (10) aus einem elektrisch leitfähigen Material, einen dritten elektrischen Kontakt, der elektrisch mit der ersten Vielzahl von Vorsprüngen verbunden ist; einen vierten elektrischen Kontakt, der elektrisch mit der zweiten Vielzahl von abgerundeten Vorsprüngen (10) verbunden ist; ein Kolben-Zylinder-System, das seinerseits umfasst: ein röhrenförmiges Element (11), das als Zylinder des Kolben-Zylinder-Systems fungiert und ein distales Ende aufweist;
einen Kolben, der: eine Stange (12) und einen Kopf (13) mit einem distalen Ende aufweist, wobei der Kolben verschiebbar in das rohrförmige Element (11) eingesetzt ist und sich frei von einer ersten Endposition, in der das distale Ende des Kopfes (13) außerhalb des rohrförmigen Elements (11) und in einem ersten Abstand (D1), gemessen parallel zur Stange, angeordnet ist, von einem distalen Ende des rohrförmigen Elements, zu einer zweiten Endposition, wobei das distale Ende des Kopfes (13) in einem zweiten Abstand (D2), gemessen parallel zur Stange, vom distalen Ende des rohrförmigen Elements angeordnet ist, der kleiner ist als der erste Abstand (D1); oder wobei das distale Ende des Kopfes (13) innerhalb des rohrförmigen Elements (11) angeordnet ist; und elastische Mittel (14), die auf den Kopf (13) einwirken und dazu ausgelegt sind, den Kolben automatisch von der ersten Endposition in die zweite Endposition zu bewegen; wobei die erste und die zweite Vielzahl von abgerundeten Vorsprüngen (10) fest mit dem distalen Ende des Arbeitskopfes verbunden sind und von diesem distal vorstehen;
wobei das röhrenförmige Element (11) fest und lösbar am Griff (3) befestigbar ist, derart, dass sich der Arbeitskopf zumindest teilweise innerhalb des Grifflörpers (3) befindet und von diesem getragen wird, wobei der erste elektrische Kontakt mit dem dritten elektrischen Kontakt und der zweite elektrische Kontakt mit dem vierten Kontakt elektrisch verbunden sind, wobei die Stange versetzt, aber im Wesentlichen parallel zur Antriebswelle (5) angeordnet ist, und in Kontakt mit der Bewegungsübertragungsfläche (7) steht, wobei das distale Ende des Kopfes (13) in der ersten Endposition außerhalb des Griffs (3) angeordnet ist, und wobei die mindestens eine Beleuchtungsvorrichtung (8), der Motor (4), der erste Generator und der zweite Spannungsgenerator gleichzeitig aktivierbar sind.

2. Automatische Vorrichtung zur Behandlung der Kopfhaut nach Anspruch 1, wobei die mindestens eine Beleuchtungseinrichtung (8) fest mit dem Griff (3) verbunden und elektrisch mit dem ersten Gleichstromgenerator verbunden ist und stromaufwärts des distalen Endes (13) des Kopfes angeordnet ist und distal ausgerichtet ist.

3. Automatische Vorrichtung zur Behandlung der Kopfhaut nach Anspruch 2, wobei die mindestens eine Beleuchtungseinrichtung (8) aus einer Vielzahl von Beleuchtungseinrichtungen (8) besteht, die entlang einer geschlossenen Bahn angeordnet sind, welche den Kolben umgibt, wenn der Arbeitskopf vom Griff (3) gehalten wird.

4. Automatische Vorrichtung zur Behandlung der Kopfhaut nach einem der vorstehenden Ansprüche, wobei die mindestens eine Beleuchtungseinrichtung (8) eine Vielzahl von LEDs ist, die Licht mit einer Wellenlänge von 630, 415 oder 850 nm emittieren können.

5. Automatische Vorrichtung zur Behandlung der Kopfhaut nach einem der vorstehenden Ansprüche, wobei jede abgerundete Erhebung (10) der ersten und/oder der zweiten Vielzahl von abgerundeten Erhebungen (10) um 0,1 bis 15 mm vom distalen Ende des Kopfes (13) vorsteht.

6. Automatische Vorrichtung zur Behandlung der Kopfhaut nach einem der vorstehenden Ansprüche, wobei der Motor (4) mit einer Drehzahl der Antriebswelle (5) von 5 bis 100 Umdrehungen pro Sekunde betreibbar ist.

7. Automatische Vorrichtung zur Behandlung der Kopfhaut nach einem der vorstehenden Ansprüche, wobei jede abgerundete Erhebung (10) der ersten und/oder der zweiten Vielzahl von abgerundeten Erhebungen (10) als Halbkugel, als Kegelstumpf mit abgerundeten Ecken oder als Kegel mit abgeschrägter Spitze ausgebildet ist.

8. Automatische Vorrichtung zur Behandlung der Kopfhaut nach einem der vorstehenden Ansprüche, wobei das distale Ende des Kopfes (13) aus einem elektrisch isolierenden Material besteht und ein zweites Gehäuse umfasst, das von dem ersten getrennt ist und jeweils mit der ersten und der zweiten Vielzahl von abgerundeten Vorsprüngen (10) in Eingriff gebracht werden kann, um diese zu stützen.

9. Automatische Vorrichtung zur Behandlung der Kopfhaut nach einem der vorstehenden Ansprüche, wobei der Mindestwinkel (α) zwischen 20° und 50° liegt.

10. Automatische Vorrichtung zur Behandlung der Kopfhaut nach einem der vorstehenden Ansprüche, wobei die mindestens eine Beleuchtungseinrichtung (8), der Motor (4) und der zweite Spannungsgenerator ebenfalls getrennt voneinander aktivierbar sind und der erste elektrische Gleichstrom auch dann erzeugt werden kann, wenn die mindestens eine Beleuchtungseinrichtung (8) und/oder der Motor (4) und/oder der zweite Spannungsgenerator nicht aktiviert sind.

## Revendications

1. Dispositif automatique (1) destiné au traitement du cuir chevelu, comprenant:
- une partie fixe (2), comprenant à son tour : un boîtier ; un premier générateur de courant continu, pouvant être activé pour générer un courant continu et disposé dans le boîtier ; un deuxième générateur de tension, qui est disposé dans le boîtier et qui peut être activé pour générer un courant ayant une forme d'onde biphasique rectangulaire ou triangulaire, d'une fréquence comprise entre 2,0 et 3,0 Hz ;
- une poignée (3) reliée électriquement au premier générateur de courant continu et au deuxième générateur de tension et comprenant, à son tour : un corps ; un moteur (4) comportant un arbre d'entraînement (5) ; - au moins un dispositif d'éclairage (8) solidaire de la poignée (3) et disposé de manière distale ou solidaire de la tête de traitement et disposé de manière distale;
dans lequel le dispositif automatique (1) pour le traitement du cuir chevelu est **caractérisé**:
• **en ce que** ledit au moins un dispositif d'éclairage (8) est activable pour émettre un rayonnement électromagnétique monochromatique cohérent non collimaté;
• **en ce que** le moteur (4) est disposé de manière proximale et, l'arbre d'entraînement (5) étant disposé de manière distale et longitudinalement par rapport au corps, un élément de transmission de mouvement (6) qui est solidaire d'une extrémité distale de l'arbre d'entraînement (5) et qui comporte une surface de transmission de mouvement (7) qui est distale et qui est inclinée, par rapport à l'arbre d'entraînement (5), d'un angle minimal (α) compris entre 5° et 85°, le moteur (4) pouvant être activé électriquement pour déplacer la surface de transmission de mouvement (7) en rotation par rapport à l'arbre d'entraînement (5);
• **en ce que** la poignée (3) comprend en outre: un élément de transmission de mouvement (6) qui est solidaire d'une extrémité distale de l'arbre d'entraînement (5) et qui présente une surface de transmission de mouvement (7) distale et inclinée, par rapport à l'arbre d'entraînement (5), d'un angle minimal (α) compris entre 5° et 85°, le moteur (4) pouvant être activé électriquement pour faire tourner la surface de transmission de mouvement (7) par rapport à l'arbre d'entraînement (5) ; un premier contact électrique ; et un deuxième contact électrique; et
• **en ce que** le dispositif automatique (1) destiné au traitement du cuir chevelu comprend en outre:
- une tête de traitement comprenant: une première pluralité de protubérances arrondies (10) réalisées en un matériau électriquement conducteur, une deuxième pluralité de protubérances arrondies (10) réalisées en un matériau électriquement conducteur, un troisième contact électrique, relié électriquement à la première pluralité de protubérances ; un quatrième contact électrique relié électriquement à la deuxième pluralité de protubérances arrondies (10) ; un système piston-cylindre, comprenant à son tour un élément tubulaire (11) qui fait office de cylindre du système piston-cylindre et qui présente une extrémité distale ;
un piston qui: comporte une tige (12) et une tête (13) présentant une extrémité distale, le piston étant inséré de manière coulissante dans l'élément tubulaire (11) avec la liberté de se déplacer depuis une première position limite, dans laquelle l'extrémité distale de la tête (13) est disposée à l'extérieur de l'élément tubulaire (11) et à une première distance (D1), mesurée parallèlement à la tige, d'une extrémité distale de l'élément tubulaire, vers une deuxième position limite, dans laquelle l'extrémité distale de la tête (13) est disposée à une deuxième distance (D2), mesurée parallèlement à la tige, de l'extrémité distale de l'élément tubulaire, qui est inférieure à la première distance (D1) ; ou dans laquelle l'extrémité distale de la tête (13) est disposée à l'intérieur de l'élément tubulaire (11) ; et des moyens élastiques (14) agissant sur la tête (13) et prédisposés pour déplacer automatiquement le piston de la première position limite à la deuxième position limite ; dans lequel les première et deuxième pluralités de protubérances arrondies (10) sont solidement fixées à l'extrémité distale de la tête de commande et font saillie distalement à partir de celle-ci;
dans lequel l'élément tubulaire (11) est solidement et réversiblement fixable à la poignée (3) de telle sorte que la tête d'actionnement se trouve au moins partiellement à l'intérieur du corps de la poignée (3) et est supportée par la poignée (3) reliant électriquement le premier contact électrique au troisième contact électrique et le deuxième contact électrique au quatrième contact, en disposant la tige décalée, mais sensiblement parallèle à l'arbre d'entraînement (5), et en contact avec la surface de transmission de mouvement (7), et en disposant l'extrémité distale de la tête (13) à l'extérieur de la poignée (3) lorsqu'elle se trouve dans la première position limite, et dans lequel le au moins un dispositif d'éclairage (8), le moteur (4), le premier générateur et le deuxième générateur de tension peuvent être activés simultanément.

2. Dispositif automatique pour le traitement du cuir chevelu selon la revendication 1, dans lequel le au moins un dispositif d'éclairage (8) est solidement fixé à la poignée (3) et connecté électriquement au premier générateur de courant continu, et est disposé en amont de l'extrémité distale (13) de la tête et orienté vers l'extrémité distale.

3. Dispositif automatique pour le traitement du cuir chevelu selon la revendication 2, dans lequel le au moins un dispositif d'éclairage (8) est constitué d'une pluralité de dispositifs d'éclairage (8) disposés le long d'un trajet en boucle qui, lorsque la tête de traitement est supportée par la poignée (3), entoure le piston.

4. Dispositif automatique pour le traitement du cuir chevelu selon l'une quelconque des revendications précédentes, dans lequel le au moins un dispositif d'éclairage (8) est constitué d'une pluralité de LED capables d'émettre une lumière ayant une longueur d'onde de 630, 415 ou 850 nm.

5. Dispositif automatique pour le traitement du cuir chevelu, selon l'une quelconque des revendications précédentes, dans lequel chaque protubérance arrondie (10) de la première et/ou de la deuxième pluralité de protubérances arrondies (10) fait saillie de manière distale à partir de l'extrémité distale de la tête (13) de 0,1 à 15 mm.

6. Dispositif automatique pour le traitement du cuir chevelu selon l'une quelconque des revendications précédentes, dans lequel le moteur (4) peut être activé avec une vitesse de rotation de l'arbre d'entraînement (5) comprise entre 5 et 100 tours par seconde.

7. Dispositif automatique pour le traitement du cuir chevelu, selon l'une quelconque des revendications précédentes, dans lequel chaque protubérance arrondie (10) de la première et/ou de la deuxième pluralité de protubérances arrondies (10) a la forme d'un hémisphère, d'un cône tronqué à angles arrondis ou d'un cône à pointe biseautée.

8. Dispositif automatique pour le traitement du cuir chevelu selon l'une quelconque des revendications précédentes, dans lequel l'extrémité distale de la tête (13) est réalisée en un matériau électriquement isolant et comprend un deuxième boîtier, séparé de l'autre, pouvant s'engager respectivement avec la première et la deuxième pluralité de protubérances arrondies (10) dans le but de les supporter.

9. Dispositif automatique pour le traitement du cuir chevelu selon l'une quelconque des revendications précédentes, dans lequel l'angle minimal (α) est compris entre 20° et 50°.

10. Dispositif automatique pour le traitement du cuir chevelu selon l'une quelconque des revendications précédentes, dans lequel au moins un dispositif d'éclairage (8), le moteur (4) et le deuxième générateur de tension peuvent également être activés séparément les uns des autres et le premier courant électrique continu peut être généré même lorsque au moins un dispositif d'éclairage (8), et/ou le moteur (4) et/ou le deuxième générateur de tension ne sont pas activés.
